# FASCICULE DE BREVET EUROPEEN

(11) **EP 1 786 828 B1**
(45) Date de publication et mention de la délivrance du brevet: **11.01.2012**
(21) Numéro de dépôt: 05805806.6
(22) Date de dépôt: 09.09.2005
(51) Int. Cl.: C07K 1/13, C12Q 1/37, C12Q 1/04

(54) **NOUVEAUX SUBSTRATS ENZYMATIQUES DÉRIVÉS DE PHÉNOXAZINONE ET LEUR UTILISATION COMME RÉVÉLATEUR DANS LA DÉTECTION DE MICROORGANISME À ACTIVITÉ PEPTIDASE**
VON PHENOXAZINON ABGELEITETE NEUE ENZYMSUBSTRATE UND DEREN VERWENDUNG ALS ENTWICKLER BEI DER DETEKTION VON MIKROORGANISMEN MIT PEPTIDASEAKTIVITÄT
NOVEL ENZYMATIC SUBSTRATES DERIVED FROM PHENOXAZINONE AND THEIR USE AS DEVELOPER IN DETECTION OF MICRO-ORGANISM WITH PEPTIDASE ACTIVITY

(30) Priorité: 10.09.2004 FR 0409593
(43) Date de publication de la demande: 23.05.2007
(73) Titulaire: BIOMERIEUX, 69280 Marcy-L'Etoile (FR)
(72) Inventeur: ANDERSON, Rosaleen Joy, Gateshead NE11 0YF (GB); GROUNDWATER, Paul William, Sunderland SR6 8LT (GB); JAMES, Arthur, Cockermouth, Cumbria CA13 9UX (GB); MONGET, Daniel, F-01150 Saint-Sorlin-en-Bugey (FR); ZAYTSEV, Andrey Victorovich, Newcastle upon Tyne NE12 8AZ (GB)
(74) Mandataire: Sprugnoli, Claude Louis Victor
(86) Numéro de dépôt international: PCT/FR2005/002249
(87) Numéro de publication internationale: WO 2006/030119

(56) Documents cités:
- WO-A-01/42491
- WO-A-96/40980
- WO-A-2004/101536
- DE-A1- 4 119 956
- DE-A1- 10 251 894
- US-A- 5 336 600
- US-B1- 6 235 493
- AGBAN A ET AL: "SYNTHESIS OF NEW FLUOROGENIC SUBSTRATES DERIVED FROM 7 AMINO-4-TRIFLUOROMETHYLCOUMARIN DETECTION OF GRAM-NEGATIVE BACTERIA GROUP A STREPTOCOCCI AND ENTEROCOCCI" ANNALES PHARMACEUTIQUES FRANCAISES, vol. 48, no. 6, 1990, pages 326-334, XP008047396 ISSN: 0003-4509
- MANAFI M ET AL: "FLUOROGENIC AND CHROMOGENIC SUBSTRATES USED IN BACTERIAL DIAGNOSTICS", MICROBIOLOGICAL REVIEWS, vol. 55, no. 3, 1991, pages 335-348, ISSN: 0146-0749
- MANAFI M ET AL: "FLUOROGENIC AND CHROMOGENIC SUBSTRATES USED IN BACTERIAL DIAGNOSTICS", MICROBIOLOGICAL REVIEWS, vol. 55, no. 3, 1991, pages 335-348, ISSN: 0146-0749

## Description

La présente invention concerne de nouveaux substrats enzymatiques chromogènes pour la détection d'activité peptidase. Ces substrats sont utilisables dans les applications comportant une étape d'hydrolyse enzymatique produisant un signal physico-chimique notamment en microbiologie, biochimie, immunologie, biologie moléculaire, histologie, etc. Comparativement aux substrats existants, la plupart uniquement fluorigènes, les substrats chromogéniques de l'invention peuvent être utilisés notamment en milieu gélifié pour la détection de micro-organismes car ils produisent une coloration ne diffusant pas dans le milieu réactionnel donc concentrée au niveau des colonies.

L'invention concerne également des milieux réactionnels contenant de tels substrats, l'utilisation des substrats ou des milieux pour la détection de bactéries à Gram négatif, à Gram positif et de levures exprimant une activité peptidase, et des procédés d'utilisation.

On appelle généralement aminopeptidase une enzyme capable de cliver par hydrolyse le groupement amide formé entre un acyle d'acide aminé et une amine primaire, et on appelle peptidase une enzyme capable de cliver par hydrolyse le groupement amide formé entre le reste acyle d'un peptide et une amine primaire. Dans la présente demande, le terme « peptidase » peut désigner, selon les cas, tant une peptidase qu'une aminopeptidase telles que définies précédemment.

Des substrats chromogéniques enzymatiques pour la détection d'activité peptidase ne diffusant pas sont décrits et déjà connus de l'état de la technique. Ainsi, de tels substrats sont couverts par les demandes de brevet WO98/04735 et WO99/38995 déposées par la Demanderesse. Néanmoins, ces substrats présentent différents inconvénients : leur synthèse est difficile, la pureté est réduite et les rendements faibles. De plus, pour une utilisation en milieux de culture, il faut définir une composition de milieu très précise pour observer une couleur.

Les seuls substrats existants qui peuvent être utilisés en milieux solides pour la détection de micro-organismes en cultures mixtes sont des substrats dérivés d'acridine et sont décrits dans la demande de brevet PCT WO2004/069804 déposée par la Demanderesse.

Des molécules dérivées de phénoxazinone sont connues pour leur capacité à produire de la fluorescence. Elles peuvent être utilisées :
- à titre d'indicateurs acido-basiques, comme décrit par exemple dans Stuzka, V. et al., 1963, Collection Czech. Chem. Commun., 28, 1399-1407 ou bien
- à titre de marqueurs fluorescents, par exemple pour suivre des modifications de conformations de protéines, comme décrit dans Nakanishi J. et al., 2001, Analytical Chemistry, 73(13), 2920-2928, ou bien par exemple pour la détection de microorganismes, comme décrit dans le brevet US5,336,600. Dans ce dernier cas, les composés décrits ont pour inconvénients qu'ils ne peuvent être utilisés que dans des milieux liquides et que la détection des microorganismes, qui se fait via la mise en évidence d'une croissance bactérienne, est effectuée par modification du potentiel redox. Il n'y a donc aucune spécificité par rapport à une activité enzymatique ni par rapport à un genre ou une espèce bactérien.

Aucun dérivé de l'aminophénoxazinone actuellement décrit, et en particulier aucun dérivé de résorufamine, n'a jamais été utilisé en tant que substrat chromogénique enzymatique utilisable en milieu gélifié.

Le document DE10251894 décrit des composés et leur utilisation en tant que substrats enzymatiques chromogènes pour la détection d'activité peptidase. Les documents US6235493, WO0142491 et Agban A et al (1990), Annales Pharmaceutiques Françaises, vol.48, no.6, pp.326-334 décrivent des tests de détection de l'activité peptidase de microorganismes. Le document WO2004101536 décrit des dérivés de phenoxazinone et leur application pour la détection de l'activité peptidase de microorganismes.
Résumé de l'invention: L'invention dans son sens le plus large est telle que définie par les revendications.

Conformément à la présente invention, il est proposé de nouveaux substrats chromogéniques enzymatiques pour la détection de microorganismes exprimant une activité peptidase. L'invention concerne également des milieux réactionnels contenant de tels substrats, ainsi que l'utilisation des substrats ou des milieux pour la détection d'activités peptidases, et des procédés d'utilisation.

En effet, la Demanderesse a trouvé de manière surprenante qu'il était possible de détecter des microorganismes exprimant une activité peptidase par l'utilisation de nouveaux dérivés de la phénoxazinone chromogéniques produisant une coloration ne diffusant pas dans le milieu réactionnel, donc concentrée au niveau des colonies, l'activité peptidase étant mise en évidence par une modification de la coloration des colonies dans le milieu de culture.

Après ensemencement des milieux réactionnels contenant les substrats de l'invention avec les microorganismes à tester, on observe des colonies incolores à blanches lorsque celles-ci ne sont pas capables d'hydrolyser le substrat. En revanche, on observe des colonies colorées lorsqu'elles sont capables d'hydrolyser le substrat de l'invention.

Les dérivés de la phénoxazinone de l'invention sont à la fois chromogéniques et fluorigènes et ont pour avantage une bonne sensibilité de détection. De plus, l'excitation et l'émission de la fluorescence se font dans le spectre visible, de sorte que la fluorescence peut être détectée à l'oeil nu et sous éclairage normal.

Selon l'invention, on entend notamment par aryle un noyau aromatique en C₆-C₁₂, notamment phényle, benzyle, 1-naphtyle ou 2-naphtyle. Il en est de même pour la partie aryle des groupements aralkyle. Ainsi le groupement alkyle du groupement aralkyle en C₆-C₁₄ est en C₂-C₈.

On entend par alkyle en Cₓ-C_{y}, un alkyle droit ou ramifié ayant de x à y atomes de carbone, dans le cas présent de 1 à 12 atomes de carbone, de 1 à 6 atomes de carbone, ou de 1 à 3 atomes de carbone ou de 2 à 8 atomes de carbone. A titre d'exemple, on peut citer le méthyle, l'éthyle, le propyle, l'iso-propyle, le butyle, l'isobutyle, le t-butyle, le pentyle, l'iso-pentyle, l'hexyle, l'heptyle, l'octyle, le nonyle, le décyle, l'undécyle et le dodécyle.

Par atome d'halogène, on entend le chlore, le brome, l'iode et le fluor.

Par hétéroatome, on entend un atome autre qu'un atome de carbone, tel que par exemple O, N ou S.

Les noyaux hétérocycliques que peuvent former R" et R"' peuvent être de toute dimension, mais de préférence ils contiennent de 5 à 7 chaînons.

Des exemples de noyau hétérocyclique comprennent le noyau morpholine, pipérazine, pipéridine, pyrrolidine et imidazolidine.

Les agents de blocage selon l'invention comprennent tout agent de blocage connu de l'homme du métier qui est capable de protéger les amines. A titre d'exemple, on peut citer le t-butoxycarbonyle (N-tBOC), le 9-fluorenyloxycarbonyle, un agent de solubilisation tel que le succinyle, ou bien un acide aminé non métabolisable, c'est-à-dire non naturel, tel que l'acide pipécolique.

Les agents de blocage ne sont pas systématiquement présents dans les composés de l'invention. Dans ce cas, c'est-à-dire lorsque les composés de l'invention ne possèdent pas d'agent de blocage (X est rien), les composés de l'invention sont sous forme de sel tel que chlorure, bromure ou trifluoroacétate.

Les acides aminés qui sont représentés par A dans la formule (I), sont tout acide aminé connu de l'homme du métier.

La présente invention a pour objet des substrats enzymatiques chromogéniques de formule (I) : 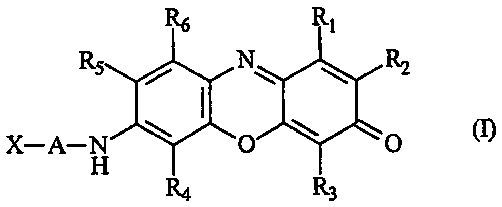 dans laquelle
- R₁ représente un atome d'hydrogène, un groupement alkyle en C₁-C₁₂, un groupement aralkyle en C₆-C₁₄, un groupement aryle, -COOH, -COOR' ou -NR"R"',
- R₂ représente un atome d'hydrogène, un atome d'halogène, un groupement alkyle en C₁-C₁₂, -COOH ou-COOR',
   étant entendu qu'au moins un parmi R₁ et R₂ est un atome d'hydrogène ou un atome d'halogène,
- R₃ représente un atome d'hydrogène, un atome d'halogène, -CN, -CONH₂, -COOR' ou -COR',
- R₄, R₅ et R₆, chacun indépendamment, représentent un atome d'hydrogène ou un groupement alkyle en C₁-C₃, étant entendu qu'au moins un parmi R₄, R₅ et R₆ est un atome d'hydrogène,
- R' représente un atome d'hydrogène ou un groupement alkyle en C₁-C₆,
- R" et R"' représentent chacun indépendamment un groupement alkyle en C₁-C₆, ou bien R" et R"', ensemble avec l'atome d'azote auquel ils sont liés, forment un noyau hétérocyclique contenant un ou plusieurs hétéroatomes,
- A représente au moins un acide aminé et
- X représente un agent de blocage ou rien.

Selon un mode de réalisation de l'invention, A représente un acide aminé ou un peptide ayant au plus 10 acides aminés dans lequel les acides aminés sont identiques ou différents. De préférence, pour une question de coût du substrat, A représente un acide aminé ou un peptide ayant au plus 4 acides aminés dans lequel les acides aminés sont identiques ou différents.

Parmi les acides aminés appropriés aux fins de l'invention, on peut citer, par exemple, l'α-alanine et la β-alanine, la leucine, la proline et la pyroglutamine.

Selon un mode de réalisation de l'invention, on préfère les composés de l'invention pour lesquels R₁ représente un groupement alkyle et R₂ représente un atome d'hydrogène. De préférence encore, R₁ représente un groupement alkyle en C₁-C₆, ou même C₃-C₆, le méthyle et le pentyle étant particulièrement préférés.

Les composés pour lesquels R₁ représente un atome d'hydrogène et R₂ représente un groupement alkyle en C₁-C₆ constituent un autre mode de réalisation de l'invention. On préfère les composés pour lesquels R₂ représente un groupement éthyle ou hexyle.

Selon encore un autre mode de réalisation, les composés de l'invention sont tels que R₃, R₄, R₅ et R₆ représentent chacun un atome d'hydrogène.

Les composés de l'invention peuvent être préparés selon le mode opératoire représenté sur le schéma 1 suivant :

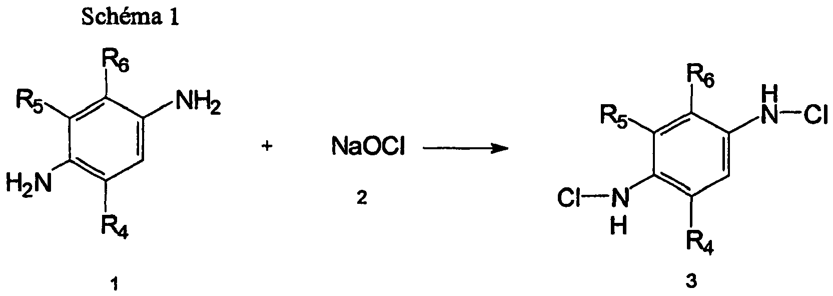

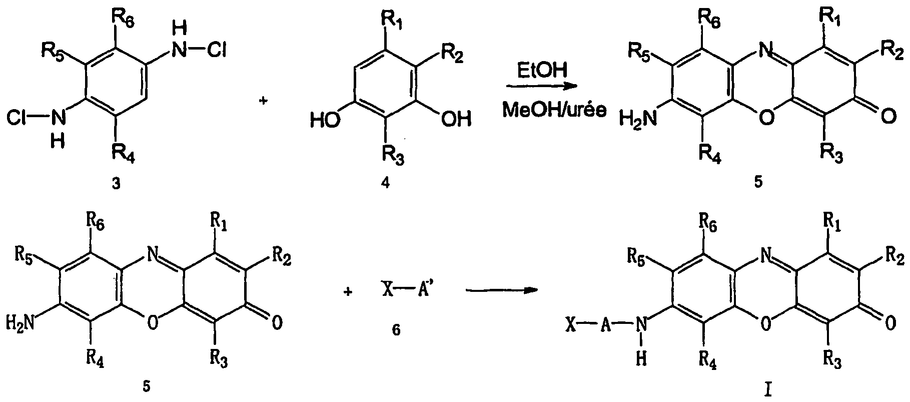

Selon le schéma 1, la dichloroimine (3) appropriée est préparée par chloration oxydative de p-phénylènediamine substituée de façon appropriée (1) en présence du composé (2) selon le protocole de Willstaetter et Mayer (1904). La dichloroimine (3) ainsi obtenue est ensuite condensée dans une solution éthanolique avec du résorcinol substitué de façon approprié (4) pour donner la résorufamine (5). La résorufamine (5) est ensuite mise à réagir avec un ou plusieurs acides aminés éventuellement protégés (6) dans un bain refroidi à environ -12°C, pour donner le composé de formule (I). Il est à noter ici que, bien entendu, lorsque A est un seul acide aminé, A' dans le composé (6) correspond à A du composé (I), mais comportant un groupement hydroxyle supplémentaire. En d'autres termes, lorsque A est un seul acide aminé, A' se termine par -C(O)OH, tandis que A est relié à NH- via -C(O)-, en perdant -OH. Lorsque A est un enchaînement d'au moins deux acides aminés, le dernier acide aminé de A' est tel que décrit précédemment, c'est-à-dire qu'il comporte, par rapport au dernier acide aminé de A, un groupement hydroxyle supplémentaire.

Ce procédé peut être utilisé pour préparer tous les composés de l'invention. Toutefois, de préférence, les composés de formule (I) pour lesquels R₁ ou R₂ sont un groupement alkyle peuvent être préparés selon le protocole décrit dans le schéma 2 ci-dessous.

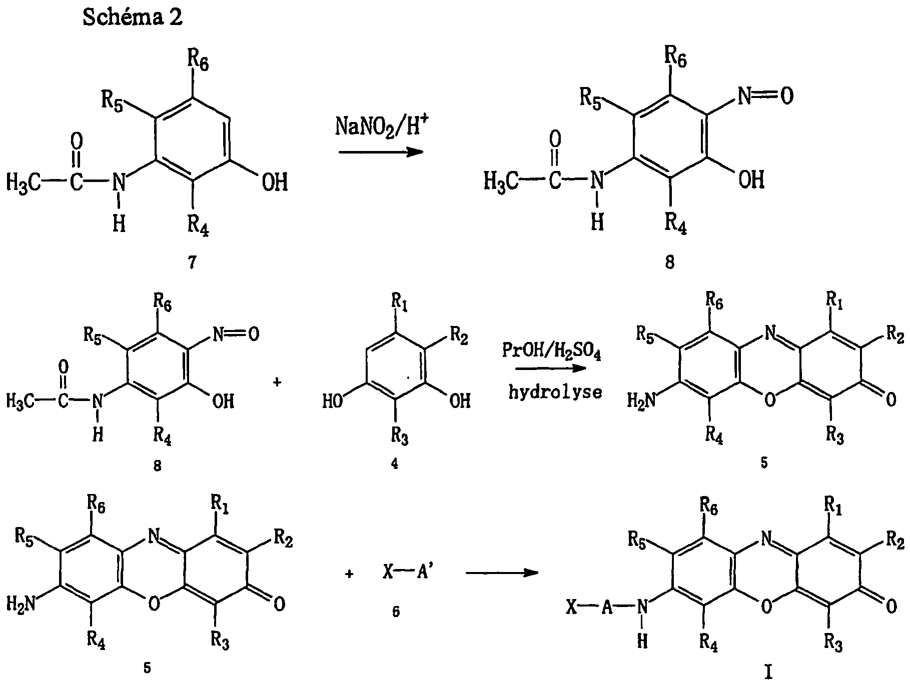

Dans le schéma 2 ci-dessus, le composé nitroso (8) est préparé par réaction d'un 3-aminophénol substitué de façon approprié (7) avec un nitrite de métal alcalin, tel que le nitrite de sodium, en présence d'acide phosphorique ou d'acide sulfurique, à une température de 0 à -3°C. Le composé nitrosoacétamidophénol (8) ainsi obtenu est ensuite mis à réagir avec un résorcinol substitué de façon appropriée (4) dans un solvant tel que propanol ou butanol, en présence d'acide sulfurique comme catalyseur acide, et un agent de cyclisation. L'acétamidophénoxazinone ainsi obtenue est désacétylée par une brève période de chauffage en présence d'acide sulfurique à 90°C, opération suivie d'un refroidissement et d'une précipitation aqueuse. La résorufamine (5) ainsi obtenue est ensuite mise à réagir avec un ou plusieurs acides aminés éventuellement protégés (6) comme indiqué dans le mode opératoire du schéma 1.

Les composés peuvent être également préparés selon le mode opératoire suivant, tel que représenté sur le schéma 3 :

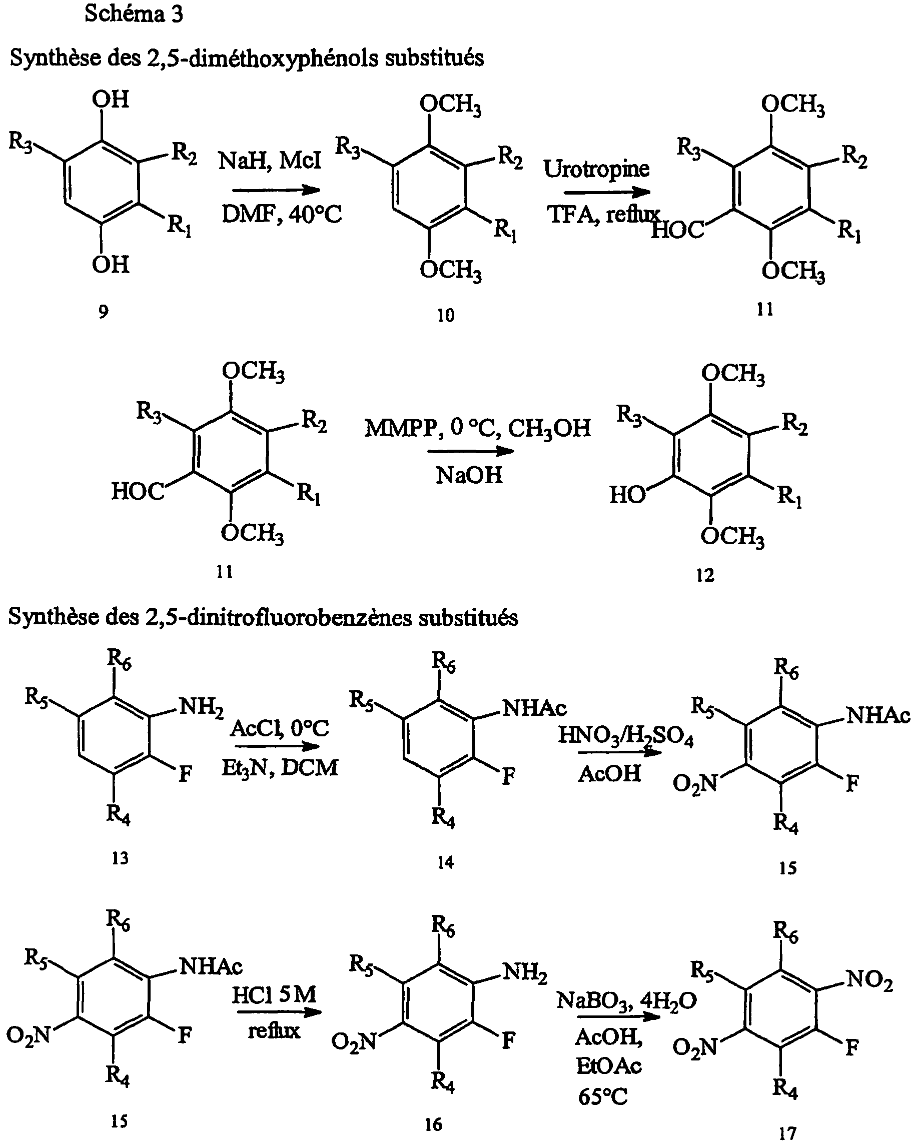

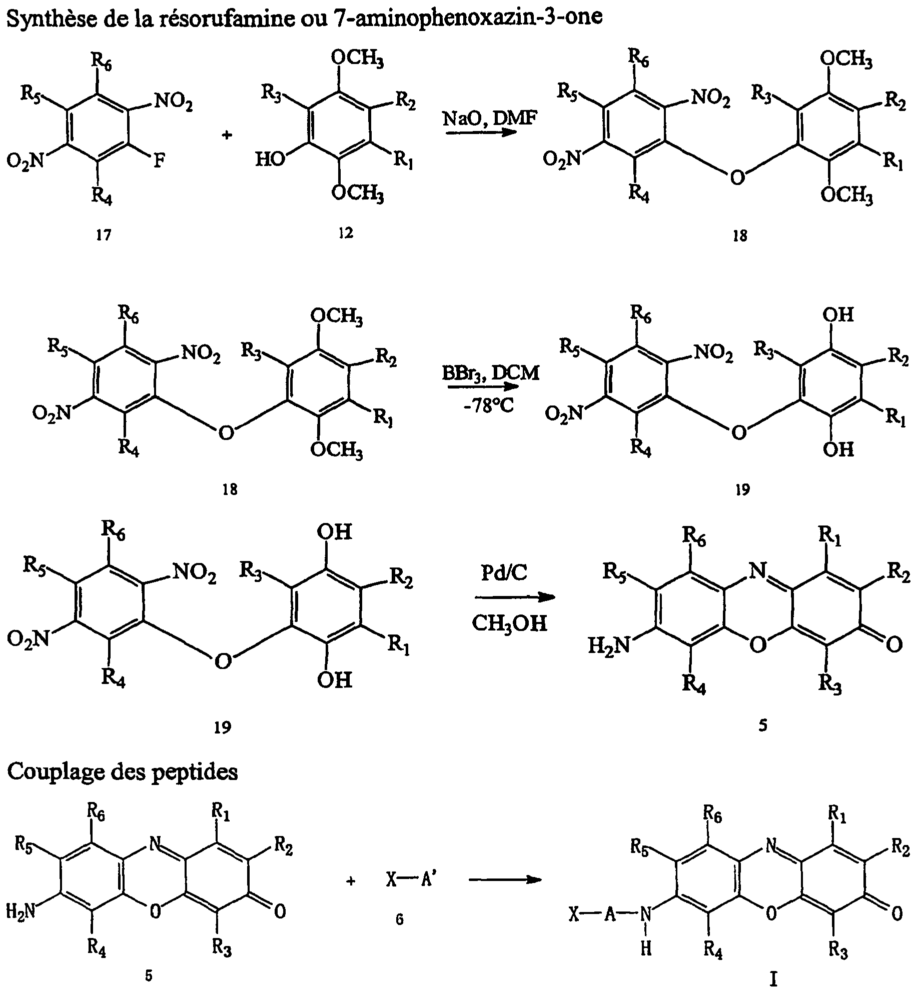

Dans le schéma 3 ci-dessus, les composés de l'invention sont préparés en 4 étapes.

Selon la première étape, le composé 2,5-diméthoxyphénol substitué de façon appropriée (12) est préparé à partir d'un composé hydroquinone (9), également substitué de façon appropriée. Ce composé (9) est mis à réagir dans du diméthylformamide (DMF) avec du NaH, puis avec de l'iodure de méthyle, réaction suivie d'une agitation à 40°C, pour donner le composé 2,5-diméthoxyphényle substitué de façon approprié (10). Ce composé est ensuite mis à réagir dans du trifluoroacétate. (TFA) avec de l'urotropine sous reflux selon la réaction de Duff (Smith WE, 1972, J Org. Chem., 37 : 3972) pour donner le 2,5-diméthoxybenzaldéhyde sustitué de façon appropriée (11). Enfin, le composé (11) est mis à réagir dans du méthanol avec du monoperoxyphtalate de magnésium à 0°C, puis dans du DMF avec du NaOH selon la réaction de Bayer-Villiger (Capecchi T., et al., 2000, J. Chem. Soc., Perkin Trans. 1, 2681) pour donner le 2,5-diméthoxyphénol substitué de façon appropriée (12).

Selon la 2^{ème} étape, le 2,5-dinitrofluorobenzène (17) est préparé à partir dune fluoroaniline (13) substituée de façon approprié. Ce composé (13) est mis à réagir à 0°C dans de la triéthylamine et du dichlorométhane (DCM) avec du chlorure d'acétyle pour donner la N-acétyl-fluoroaniline substituée de façon appropriée (14). Ce composé est ensuite mis à réagir avec un mélange d'acide sulfurique concentré et d'acide nitrique dans de l'acide acétique pour donner le composé nitroaniline (15) substitué de façon appropriée. Ce composé (15) est porté au reflux avec de l'acide chlorhydrique pour donner le composé nitroaniline (16) qui lui-même est mis à réagir dans l'acide acétique avec du perborate sodique à 65°C pour donner le composé (17).

Selon la 3^{ème} étape, la résorufamine (5) est obtenue par réaction du 2,5-diméthoxybenzène (12) obtenu dans la 1^{ère} étape et du 2,5-dinitrofluorobenzène (17) obtenu dans la 2^{ème} étape comme suit : les composés (12) et (17) sont mis à réagir dans du DMF avec du NaH à la température ambiante pour donner le biaryléther (18) substitué de façon appropriée. Ce composé (18) est ensuite mis à réagir dans du DCM avec du BBr₃ pour donner le dihydroxybiaryléther (19) qui est lui-même mis à réagir en présence du catalyseur Pd/C et du méthanol pour donner la résorufamine (5).

Enfin, selon la dernière étape, la résorufamine (5) ainsi obtenue est ensuite mise à réagir avec un ou plusieurs acides aminés éventuellement protégés (6) comme indiqué dans le mode opératoire du schéma 1.

Dans les modes opératoires ci-dessus, les réactifs de départ (composés (1), (2), (4), (6), (7), (9) et (13)) sont disponibles dans le commerce, notamment chez Sigma.

L'invention a également pour objet un milieu réactionnel comprenant au moins un substrat chromogénique enzymatique de formule (I) telle que définie précédemment, seul ou en combinaison avec au moins un autre substrat enzymatique spécifique d'une activité enzymatique différente de l'activité peptidase détectée par le substrat selon l'invention.

En effet, lorsque des microorganismes exprimant une activité peptidase sont ensemencés dans ou sur un milieu réactionnel contenant les composés de l'invention, il se produit une coloration ne diffusant pas dans ou sur le milieu réactionnel, et donc concentrée au niveau des colonies.

Par milieu réactionnel selon l'invention, on entend un milieu permettant le développement d'au moins une activité enzymatique d'au moins un microorganisme.

Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture, ce qui constitue un mode de réalisation particulier de l'invention.

Le milieu réactionnel peut être solide, semi-solide ou liquide. Par milieu solide, on entend par exemple un milieu gélifié.

L'agar est le milieu traditionnel solide en microbiologie pour la culture des microorganismes, mais il est possible d'utiliser de la gélatine, de l'agarose ou un autre gélifiant. Un certain nombre de préparation sont disponibles dans le commerce, comme par exemple la gélose Columbia, la gélose Trypcase-soja, la gélose Mac Conkey, la gélose Sabouraud ou plus généralement celles décrites dans le Handbook of Microbiological Media (CRC Press).

De préférence, lorsque le milieu réactionnel est également un milieu de culture, il est sous forme gélifiée.

La quantité d'agar dans le milieu réactionnel est de 2 à 40g/l. Pour les milieux solides, la quantité d'agar est de préférence de 9 à 25g/l, de préférence encore de 12 à 14g/l, et pour les milieux semi-solides, la quantité d'agar est de préférence de 2 à 6g/l.

Les substrats enzymatiques de l'invention sont utilisables dans une large gamme de pH, notamment entre pH 5,5 et 10.

La concentration de substrat enzymatique de l'invention dans le milieu réactionnel est comprise entre 0,01 et 1 g/l, de préférence entre 0,025 et 0,40 g/l et, avantageusement, elle est de 0,05 g/l. En effet, à cette concentration de substrat, on obtient un meilleur contraste de coloration.

Le milieu réactionnel peut comprendre au moins un autre substrat spécifique d'une activité enzymatique différente de l'activité peptidase détectée par le substrat selon l'invention. L'hydrolyse enzymatique du ou des autres substrats génère un signal détectable, différent du signal détecté par le substrat de l'invention, comme par exemple des produits colorés ou fluorescents différents, pour permettre la mise en évidence comme la détection et/ou l'identification et/ou la quantification d'un ou plusieurs microorganismes:

A titre d'autre substrat spécifique, on peut citer les substrats de type indoxyle tels que le 5-bromo-4-chloro-3-indoxyle-β-D-glucoside (BIOSYNTH) ou le 5-bromo-6-chloro-3-indoxyle-β-D-galactoside (BIOSYNTH), ou tout autre substrat utilisé dans la détection des microorganismes.

La concentration de l'autre substrat enzymatique spécifique est généralement comprise entre 0,01 et 2 g/l. L'homme du métier pourra déterminer facilement une telle concentration en fonction du substrat utilisé.

Le milieu réactionnel peut également comprendre un ou plusieurs éléments en combinaison, tels que des acides aminés, des peptones, des hydrates de carbone, des nucléotides, des minéraux, des vitamines, des antibiotiques, des tensioactifs, des tampons, des sels de phosphate, d'ammonium, de sodium, de métaux. Des exemples de milieux sont décrits dans les demandes de brevet de la Demanderesse, EP 656 421 et WO99/09 207.

Les substrats enzymatiques et milieux réactionnels de l'invention sont donc utiles dans le diagnostic de microorganismes à activité peptidase.

Ainsi, la présente invention a également pour objet l'utilisation d'un substrat enzymatique chromogénique de formule (I), ou d'un milieu réactionnel tel que défini précédemment, pour la détection et/ou l'identification et/ou la quantification *in vitro* des micro-organismes exprimant au moins une activité peptidase.

Elle concerne également un procédé pour la détection et/ou l'identification et/ou la quantification des micro-organismes exprimant au moins une activité peptidase, caractérisé en ce qu'il consiste à :
- disposer d'un milieu réactionnel, tel que défini précédemment,
- ensemencer le milieu avec un échantillon biologique à tester,
- laisser incuber, et
- révéler la présence d'au moins une activité peptidase seule ou en combinaison avec au moins une autre activité enzymatique différente de cette même activité peptidase.

Les étapes d'ensemencement et d'incubation sont largement connues de l'homme du métier.

Par exemple, la température d'incubation est généralement comprise entre 20 et 55°C, le plus souvent entre 25 et 45°C, les températures de 30, 35 et 37°C étant les températures d'incubation les plus fréquemment utilisées. S'agissant de l'atmosphère d'incubation, elle est indifféremment anaérobie ou aérobie.

La révélation est mise en oeuvre à l'oeil nu par visualisation d'un changement de coloration ne diffusant pas dans le milieu réactionnel, donc concentrée, au niveau des colonies.

A titre de microorganismes qui peuvent être diagnostiqués grâce aux substrat enzymatique de l'invention, on peut citer les bactéries à Gram négatif, à Gram positif et les levures.

A titre de bactéries à Gram négatif, on peut citer les bactéries des genres suivants : *Pseudomonas, Escherichia, Salmonella, Shigella, Enterobacter, Klebsiella, Serratia, Proteus, Campylobacter, Haemophilus, Morganella, Vibrio, Yersinia, Acinetobacter, Branhamella, Neisseria, Burkholderia, Citrobacter, Hafnia, Edwardsiella* et *Legionella.*

A titre de bactéries à Gram positif, on peut citer les bactéries des genres suivants : *Enterococcus, Streptococcus, Staphylococcus, Bacillus, Listeria, Clostridium, Mycobacteria* et *Corynebacteria.*

Des exemples de levures comprennent les levures des genres suivants : *Candida, Cryptococcus, Saccharomyces* et *Trichosporon.*

Les substrats de l'invention sont particulièrement appropriés pour la détection des bactéries à Gram négatif car on obtient à la fois une croissance des microorganismes et une coloration nette des colonies.

En particulier, les substrats chromogéniques de l'invention, dans lesquels A est la L-alanine, ont pour avantage qu'ils permettent de différencier nettement les bactéries à Gram négatif des bactéries à Gram positif.

Ainsi un autre objet de l'invention consiste en un procédé pour la différentiation chez des bactéries de leur appartenance aux germes à Gram positif ou aux germes à Gram négatif, caractérisé en ce qu'il consiste à :
- disposer d'un milieu réactionnel, tel que défini précédemment et dans lequel le substituant A du substrat chromogénique est la L-alanine,
- ensemencer le milieu avec un échantillon biologique à tester,
- laisser incuber, et
- révéler la présence d'au moins une variation de coloration synonyme de la présence de germe(s) à Gram négatif.

Les substrats chromogéniques de l'invention dans lesquels A est la β-alanine ou la pyroglutamine ont pour avantage qu'ils permettent de distinguer *Pseudomonas aeruginosa* des autres genres, et également des autres espèces de *Pseudomonas.*

Ainsi, un autre objet de l'invention consiste en un procédé pour la détection de *Pseudomonas aeruginosa,* caractérisé en ce qu'il consiste à :
- disposer d'un milieu réactionnel, tel que défini précédemment et dans lequel le substituant A du substrat chromogénique est la β-alanine ou la pyroglutamine,
- ensemencer le milieu avec un échantillon biologique à tester,
- laisser incuber, et
- révéler la présence d'au moins une variation de coloration synonyme de la présence de germe(s) de *Pseudomonas aeruginosa.*

Les échantillons biologiques à analyser sont tout échantillon clinique comme un prélèvement de salive, de sang, d'urine, de selles ou tout autre échantillon dont l'analyse peut aider un clinicien à poser un diagnostic. L'échantillon peut également être un échantillon de produit issu ou de base de l'industrie alimentaire et/ou pharmaceutique dans lequel il faut soit garantir l'absence de microorganismes pathogènes, soit dénombrer une flore contaminante, soit détecter des microorganismes spécifiques.

L'invention sera mieux comprise à l'aide des exemples suivants donnés à titre illustratif et non limitatif.

### Exemple 1 : Synthèse de la 7-N-(N'-t-butoxycarbonyl-L-alanyl)amino-2-chloro-1-pentyphénoxazin- -3-one et de la 7-N-(N'-t-butoxycarbonyl-L-alanyl)-amino-1-pentylphénoxazin -3-one

On a préparé de la 1,4-dichlorobenzoquinonediimine par chloration de 1,76 g (10 mmol) de p-phénylènediamine et on l'a dissoute en chauffant en présence de méthanol anhydre (50 ml) et 9 g d'urée ajoutés à la solution agitée. On a ensuite ajouté 1,8 g (10 mmol) de 5-pentylrésorcinol à la solution à 40-50°C et, après dissolution complète, le mélange réactionnel a été porté au reflux avec précaution pour éviter une exothermie excessive. On a poursuivi le chauffage pendant 1,5 h, après quoi on a ajouté lentement le mélange réactionnel refroidi à un mélange glace/eau bien agité, contenant de l'ammoniac. On a recueilli le précipité par filtration par aspiration et on l'a lavé avec de l'eau, puis on l'a séché à l'air pour obtenir 2,2 g de produit brut constitué d'un mélange des composés du titre, le produit non chloré étant majoritaire.

On a ajouté goutte à goutte, tout en agitant, de l'acide acétique (30%, 200 cm³) d'un flacon à 2 cols, à une solution constituée d'environ 5 g de borhydrure de sodium et 200 mg d'hydroxyde de sodium dissous dans 200 cm³ d'eau. On a fait passer du gaz hydrogène dans un flacon à 3 cols dans lequel le mélange de 7-amino-2-chloro-1-pentylphénoxazin-3-one et de 7-amino-1-pentylphénoxazin-3-one (0,564 g) a été dissous dans du diméthylformamide anhydre (15 cm³ en chauffant, puis en refroidissant) et la solution a été diluée avec du tétrahydrofurane (THF) anhydre (15 cm³). Un catalyseur Pd/C (5%, 200 mg) a été ajouté à la solution. On a fait barboter doucement de l'hydrogène gazeux dans la solution et on a poursuivi longtemps après la réduction apparente (environ 1h). La réduction a été mise en évidence par remplacement de la couleur pourpre de la solution par une couleur gris-vert. On a ainsi obtenu une solution de résorufamine.

Dans un flacon séparé, on a dissous 0,756 g (4,0 mmol) de *N-t-Boc*-L-alanine et 0,408 g (4,0 mmol) de N-méthylmorpholine dans du THF anhydre (10 cm³), on a refroidi la solution à -20 °C et on a ajouté 0,56 cm³ (4,0 mmol) de chloroformiate d'isobutyle tout en agitant. On a agité le mélange à -20 °C pendant 30 min supplémentaires, après quoi on a introduit à -10 °C le mélange dans la solution de résorufamine en agitation, tout en faisant barboter de l'hydrogène gazeux. Après 15 min, on a stoppé l'admission d'hydrogène, on a scellé le système et le mélange réactionnel a été agité toute la nuit à température ambiante. On a filtré le mélange réactionnel et on a fait évaporer le solvant sous pression réduite, on a dissous le solide résiduel dans du dichlorométhane (DCM), on a filtré et on a lavé la solution de DCM avec du NaHCO₃ (5%, 2 fois 50 cm³) et de l'eau (50 cm³). On a séché la phase organique avec du MgSO₄, on a filtré et on a concentré pour obtenir un résidu constitué des deux produits du titre. On les a purifiés par chromatographie sur colonne de silice, en éluant avec un mélange d'essence et d'acétate d'éthyle (7:3). Le premier spot correspond à la 7-N-(*N*'-*t-butoxycarbonyl*-L-alanyl)amino-2-chloro-1-pentyl-phénoxazin- -3-one sous forme de solide orange et le deuxième spot correspond à la 7-N-*(N'-t-butoxycarbonyl-L-alanyl)-amino-1-pentylphénoxazin-3-one* sous forme de solide brun.

### Exemple 2 : Synthèse de la 7-N-(N'-t-butoxycarbonyl-β-alanyl)amino-2-chloro-1-pentylphénoxazin- -3-one et de la 7-N-(N'-t butoxvcarbonyl-β-alanyl)-amino-1-pentylphénoxazin-3-one

On a répété le mode opératoire décrit dans l'exemple 1 ci-dessus, à ceci près qu'on a utilisé de la *N*-*t*-*Boc*-β-alanine à la place de la *N-t-Boc*-L alanine.

Pour récupérer les composés du titre, on a procédé à une chromatographie sur colonne de silice en éluant avec un mélange d'essence/acétate d'éthyle (6:4). Le premier spot correspond à la 7-N-(*N*'-*t-butoxycarbonyl*-β-alanyl)amino-2-chloro-1-pentylphénoxazin-3-one sous forme de solide orange et le deuxième spot correspond à la 7-N-(*N*'-*t*-*butoxycarbonyl*-β-alanyl)-amino-1-pentylphénoxazin-3-one sous forme de solide orange.

### Exemple 3 : Déprotection des dérivés aminés

Pour ce faire, on a dissous dans 2 cm³ de TFA (trifluoroacétate) les composés obtenus dans les exemples 1 et 2 selon les proportions suivantes : 0,10 g (0,21 mmo 1) pour la 7-N-(*N*'-*t*-*butoxycarbonyl*-L-alanyl)amino-2-chloro-1-pentylphénoxazin--3-one et 80 mg (0,18 mmol) chacun pour les 7-N-(*N*'-*t*-*butoxycarbonyl*-L-alanyl)amino-1-pentylphénoxazin-3-one, 7-N-(N'-t-butoxycarbonyl-β-alanyl)amino-2-chloro-1-pentyl-phénoxazin-3-one et 7-N-(*N*'-t-butoxycarbonyl-β-alanyl)amino-1-pentylphénoxazin-3-one.

On a conservé le mélange pendant 15 min à la température ambiante. On a enregistré la progression de la réaction par chromatographie sur couche mince jusqu'à ce qu'aucun matériau supplémentaire de départ n'ait été mis en évidence. On a fait évaporé sous vide le TFA et on a complètement lavé le résidu avec de l'éther et on a séché pour obtenir les différents composés sous forme de sel de trifluoroacétate (solide brun) selon les rendements suivants : 0,095 g (92%) pour la 7-N-(*N*'-*t-butoxycarbonyl-*L-alanyl)amino-2-chloro-1-pentylphénoxazin-3-one et 80 mg (97%) chacun pour les 7-N-(*N*'-*t*-*butoxycarbonyl*-L-alanyl)amino-1-pentylphénoxazin-3-one, 7-N-(*N*'-*t-butoxy-carbonyl*-β-alanyl)amino-2-chloro-1-pentylphénoxazin-3-one et 7-N-(*N*'-*t*-*butoxy-carbony*l-β-alanyl)amino-1-pentylphénoxazin-3-one.

### Exemple 4: Synthèse de la 7-N-(L-pyroglutamyl)amino-2-chloro-1-pentyl-phénoxazin-3-one et de la 7-N-(L-pyroglutamyl)-amino-1-pentylphénoxazin-3-one

On a répété le mode opératoire décrit dans l'exemple 1 ci-dessus, à ceci près qu'on a utilisé 0,516 g d'acide L-pyroglutamique à la place de la *N-t-Boc*-L alanine.

Pour récupérer les composés du titre, on a procédé à une chromatographie sur colonne de silice en éluant avec un mélange de DCM/MeOH (95:5). Le premier spot correspond à la 7-N-(L-pyroglutamyl)amino-2-chloro-1-pentyl-phenoxazin-3-one sous forme de solide brun et le deuxième spot correspond à la 7-N-(L-pyroglutamyl)-amino-1-pentyl-phénoxazin-3-one sous forme de solide brun.

### Exemple 5 : Synthèse de la 7-N-(N'-t-butoxycarbonyl-β-alanyl)amino-1,2-diméthyl-phénoxazin-3-one et de la 7-N-(N'-t butoxycarbonyl-β-alanyl)amino-1,2,4-triméthyl-phénoxazin-3-one

### 5.1. Procédure générale pour la préparation de diméthoxybenzènes

Dans un flacon sec à fond rond et à 2 cols muni d'un condenseur, d'un aimant droit d'agitation et d'un tube de protection au chlorure de calcium, on a dissous de l'hydroquinone (1 équivalent molaire) dans 50 ml de diméthylformamide (DMF) anhydre et on a ajouté 2,2 équivalents molaires de NaH en petites quantités. Après ajout de la base et quand l'H₂ a cessé d'évoluer, on a ajouté goutte à goutte 4 équivalents molaires d'iodure de méthyle en 15-20 min. A la fin de l'addition, on a agité le mélange réactionnel à 40°C pendant 2 heures. On a ajouté 200 ml d'eau salée dans le flacon et on a extrait le mélange résultant avec du diéthyléther (3 fois 50 ml). On a lavé les couches organiques combinées avec de l'eau (2 fois 50 ml) et on a séché sur du MgSO₄. On a évaporé le solvant sous pression réduite et on a soumis le résidu à une chromatographie sur colonne.

### 5.1.1 1,4-Diméthoxy-2,3-diméthylbenzène

On a procédé comme décrit dans le point 5.1 ci-dessus à partir de 1,957 g (0,01416 mol) de 2,3-diméthylhydroquinone. On a isolé le produit sous forme de solide blanc (2,27 g, 80%) en utilisant un mélange 95:5 d'essence minérale légère:diéthyléther.

### 5.1.2. 1,4-Diméthoxy-2,3,5-triméthylbenzène

On a procédé comme décrit dans le point 5.1 ci-dessus à partir de 2,175 g (0,41429 mol) de 2,3,5-triméthylhydroquinone. On a isolé le produit sous forme d'huile incolore (2,367 g, 92%).

### 5.2. Formylation des diméthoxybenzènes par la réaction de Duff

On a dissous 1 équivalent de diméthoxybenzène dans 20 ml de TFA et on a ajouté 1,05 équivalent d'urotropine à la solution résultante. On a porté au reflux le mélange réactionnel pendant 2 heures dans des conditions anhydres. On a évaporé le TFA sous pression réduite, on a dissous le résidu dans 100 ml d'éther et on a lavé la solution organique avec de l'eau (3 fois 50 ml), puis on l'a séchée sur du MgSO₄. On a évaporé le solvant et on a soumis le résidu à une chromatographie sur colonne, en effectuant une élution avec un mélange 80:20 d'essence minérale légère (60-80°C):diéthyléther.

### 5.2.1. 2.5-Diméthoxy-3,4-diméthylbenzaldéhyde

On a procédé comme décrit dans le point 5.2 ci-dessus à partir de 2,270 g (0,01366 mol) de 1,4-diméthoxy-2,3-diméthylbenzène. On a isolé le produit du titre sous la forme d'un solide blanc (1,18 g, 44%).

### 5.2.2 2,5-Diméthoxy-3,4,6-triméthylbenzaldéhyde

On a procédé comme décrit dans le point 5.2 ci-dessus à partir de 2,274 g (0,01262 mol) de 1,4-diméthoxy-2,3,5-triméthylbenzène. On a isolé le produit du titre sous la forme d'un solide jaune (1,21 g, 46%).

### 5.3 Procédure générale pour la préparaton de phénols en utilisant l'oxydation de Bayer- Villiger

On a dissous 0,033 mol de diméthoxybenzaldéhyde dans 50 ml de méthanol et on a ajouté goutte à goutte à une suspension de monoperoxyphtalate de magnésium (MMPP) (0,018 mol) dans 50 ml de méthanol tout en conservant le mélange réactionnel à 0°C. A la fin de l'addition, on a agité le mélange réactionnel à la température ambiante pendant 4 heures. On a hydrolyse l'ester résultant dans des conditions basiques en utilisant 50 ml de NaOH 1M. Après 1 heure, on a retiré trois quarts du méthanol sous pression réduite, on a neutralisé la base en excès avec du HCl 1M et on a ajusté le pH à 3. On a extrait le phénol dans de l'acétate d'éthyle (3 fois 50 ml), on a séché les couches organiques combinées sur MgSO₄, on a évaporé le solvant et on a soumis le résidu à une chromatographie sur colonne.

### 5.3.1. 2,5-Diméthoxy-3,4-diméthylphénol

On a procédé comme décrit dans le point 5.3 ci-dessus à partir de 1,126 g (5,797 mmol) de 2,5-diméthoxy-3,4-diméthybenzaldéhyde. On a isolé le produit du titre sous la forme d'une huile jaune (0,239 g, 23%) en utilisant, comme éluant, un mélange de 60:40 d'essence minérale légère (60-80°C):diéthyléther.

### 5.3.2. 2,5-Diméthoxy-3,4,6-triméthylphénol

On a procédé comme décrit dans le point 5.3 ci-dessus à partir de 1,205 g (5,786 mmol) de 2,5-diméthoxy-3,4,6-diméthybenzaldéhyde. On a isolé le produit du titre sous la forme d'un solide blanc (0,856 g, 75%) en utilisant, comme éluant, un mélange de 75:25 d'essence minérale légère (60-80°C):diéthyléther.

### 5.4. Procédure générale de préparation des biaryléthers

On a dissous 1 équivalent molaire de phénol approprié dans 10 ml de DMF anhydre et on a ajouté 1,1 équivalent molaire de NaH en petites quantités. Après évolution totale du gaz, on a agité la solution résultante de phénolate de sodium à la température ambiante pendant 15 minutes. On a ajouté goutte à goutte au flacon 1 équivalent molaire d'une solution de 2,5-dinitrofluorobenzène dans 5 ml de THF anhydre et on a agité le mélange réactionnel pendant 2 heures. A la fin, on a versé le contenu du flacon dans 50 ml d'eau, on a extrait avec de l'éther (3 fois 50 ml) et on a séché les couches organiques combinées sur du MgSO4. On a retiré le solvant sous pression réduite et on a soumis le résidu à une chromatographie sur colonne.

### 5.4.1 1-(2',5'-dinitrophénoxy)-3,4-diméthylbenzène

On a procédé comme décrit dans le point 5.4 ci-dessus à partir de 0,293 g (1,575 mmol) de 2,5-dinitrofluorobenzène et de 0,287 g (1,575 mmol) de 2,5-diméthoxy-3,4-diméthylphénol. On a obtenu le produit du titre sous la forme d'un solide orange (0,415 g, 76%) après chromatographie sur colonne en utilisant, comme éluant, un mélange 85:15 d'essence minérale légère (60-80°C):acétate d'éthyle.

### 5.4.2 1-(2',5'-dinitrophénoxy)-3,4,6-triméthylbenzène

On a procédé comme décrit dans le point 5.4 ci-dessus à partir de 0,812 g (4,362 mmol) de 2,5-dinitrofluorobenzène et de 0,856 g (4,362 mmol) de 2,5-diméthoxy-3,4,6-triméthylphénol. On a obtenu le produit du titre sous la forme d'un solide jaune (1,412 g, 89%) après chromatographie sur colonne en utilisant, comme éluant, un mélange 75:25 d'essence minérale légère (60-80°C):diéthyléther.

### 5.5. Procédure générale pour la déprotection des hydroquinone-méthyl-éthers

On a dissous 1 équivalent molaire de diméthylaryléther dans 30 ml de DCM anhydre et on a refroidi à -78°C. On a ajouté goutte à goutte 2,5 équivalents molaires BBr₃ 1M dans de l'hexane à la solution refroidie d'éther et on a agité le produit réactionnel à -78°C pendant 30 min. On l'a ensuite chauffé à la température ambiante et on l'a agité jusqu'à la fin de la réaction (suivie par chromatographie sur couche mince). On a dilué le mélange réactionnel avec 10 ml de méthanol à 0°C et on l'a versé dans 50 ml d'eau. On a séparé la couche organique et on a lavé la couche aqueuse avec de l'acétate d'éthyle (3 fois 25 ml). On a séché les couches organiques combinées sur

du MgSO₄. On a retiré le solvant et on a soumis le résidu à une chromatographie sur colonne.

### 5.5.1. 1-(2',5'-dinitrophénoxy)-3,4-diméthyl-2,5-dihydroxybenzène

On n'a pas isolé le composé du titre, mais il a été formé, puis réduit et cyclisé directement selon une réaction dans un même récipient (voir le point 5.6.1. ci-dessous).

### 5.5.2. 1-(2',5'-dinitrophénoxvy)-3,4,6-triméthyl-2,5-dih,droxybenzène

On a procédé comme décrit dans le point 5.5 ci-dessus à partir de 0,626 g (1,728 mmol) de 1-(2',5'-dinitrophénoxy)-2,5-diméthoxy-3,4,6-diméthybenzaldéhyde. On a isolé le produit du titre sous la forme d'un solide orange (0,435 g, 75%) en utilisant, comme éluant, un mélange de 70:30 d'essence minérale légère (60-80°C):diéthyléther.

### 5.6. Procédure générale pour préparer les 7-aminophénoxazin-3-ones

On a dissous 1,3 mmol de dihydroxybiaryléther dans 5 ml de méthanol et on a ajouté du catalyseur Pd/C 5% (10% poids/poids) à la solution. On a agité le mélange réactionnel à la température ambiante dans l'appareil d'hydrogénation sous atmosphère d'hydrogène pendant 4 heures. On a ajouté de la silice au flacon (en quantité suffisante en vue de la charge de résidu à introduire dans la chromatographie sur colonne) et on a agité le mélange vigoureusement pendant 4 heures supplémentaires sous accès libre d'air. A la fin de l'oxydation, on a retiré le solvant et on a soumis le résidu à une chromatographie sur colonne en utilisant un gradiant d'éluant, en partant d'un mélange 50:50 d'essence minérale légère (60-80°C):acétate d'éthyle, puis en passant à un mélange 25:75 puis un mélange 0:100 des mêmes solvants. Enfin, on a utilisé un mélange 90:10 d'acétate d'éthyle:méthanol comme éluant.

### 5.6.1. 7-Amino-1,2-diméthylphénoxazin-3-one

On a procédé comme décrit dans le point 5.6 ci-dessus en partant de 0,266 g (0,7636 m mol) de 1-(2',5'-dinitrophénoxy)-3,4-diméthyl-2,5-dihydroxybenzène selon une réaction dans le même récipient. On a obtenu le produit du titre sous forme d'un solide brun-rouge (0,125 g, 68%).

### 5.6.2. 7-Amino-1,2,4-triméthylphénoxazin-3-one

On a procédé comme décrit dans le point 5.6 ci-dessus en partant de 0,435 g (1,3013 m mol) de 1-(2',5'-dinitrophénoxy)-3,4,6-triméthyl-2,5-dihydroxybenzène. On a obtenu le produit du titre sous forme d'un solide brun-rouge (0,237 g, 72%).

### 5.7 Procédure générale de couplage depeptide aux 7-aminophénoxazin-3-ones

On a dissous 0,4 mmol de 7-aminophénoxazin-3-one dans 5 ml de DMF anhydre dans un petit flacon à fond rond contenant un aimant droit d'agitation et on a ajouté 0,010 g de catalyseur Pd/C 5% à la solution. On a placé le flacon dans un appareil d'hdrogénation à la température ambiante et on a maintenu une atmosphère d'hydrogène tout en agitant le mélange réactionnel pendant 1 heure. On a pu confirmer la réduction complète par remplacement d'une couleur pourpre intense de la solution en couleur gris vert. Dans un flacon séparé, on a dissous 0,089 g (0,4719 mmol) de N-t-Boc-Alanine, 0,072 g (0,4719 mmol) de hydroxybenzotriazole (HOBt) et 0,07 ml (0,4719 mmol) de diisopropylcarbodiimide (DIC) dans 5 ml de DCM anhydre et on a agité le mélange réactionnel à la température ambiante pendant 1 heure. Après cette période, on a introduit le contenu du second flacon dans le premier flacon (qui contenait la forme réduite de la 7-aminophénoxazin-3-one) par l'intermédiaire d'une seringue sous atmosphère inerte. La présence d'oxygène de l'air a été évitée en raison de la très rapide oxydation du réactif. On a agité le mélange pour 20 heures supplémentaires à la température ambiante. Le mélange réactionnel a été filtré au travers de célite et le solvant a été évaporé. On a redissous le résidu dans 20 ml d'acétate d'éthyle, on a lavé la couche organique avec 20 ml d'HCl 1M, 20 ml de Na₂CO₃ 10% et 20 ml d'eau. On l'a séchée sur du MgSO₄, on l'a filtrée et on l'a évaporée sous pression réduite pour obtenir un résidu qu'on a purifié par chromatographie sur colonne en utilisant un mélange 30:70 d'essence minérale légère (60-80°C):acétate d'éthyle comme éluant.

### 5.7.1 7-N-(N'-t-butoxycarbonyl-β-alanyl)amino-1,2-diméthylphénoxazin-3-one

On a procédé comme décrit dans le point 5.7. ci-dessus à partir de 0,110 g (0,4578 mmol) de 7-amino-1,2-diméthylphénoxazin-3-one. On a obtenu le produit du titre sous forme de solide brun-rouge (0,104 g, 55%).

### 5.7.2 7-N-(N'-t-butoxycarbonyl-β-alanyl)amino-1,2,4-triméthylphénoxazin-3-one

On a procédé comme décrit dans le point 5.7. ci-dessus à partir de 0,100 g (0,3933 mmol) de 7-amino-1,2,4-triméthylphénoxazin-3-one. On a obtenu le produit du titre sous forme de solide orange (0,113 g, 68%).

### 5.8. Déprotection du gouvernant N-t-butoxycarbonyle

On a dissous 0,2 mmol du composé protégé par le *N-t-butoxycarbonyle* correspondant dans 3 ml de DCM anhydre et on a ajouté 1 ml de TFA à la solution. On a agité le mélange réactionnel à la température ambiante jusqu'à la fin de la réaction (suivie par chromatographie sur couche mince). On a fait évaporer le solvant et le TFA en excès sous pression réduite et on a purifié le résidu par chromatographie sur colonne courte en utilisant un gradient d'éluant, en partant d'un mélange 50:50 d'essence minérale légère (60-80°C):acétate d'éthyle, puis en passant à un mélange 0:100 des mêmes solvants. Enfin, on a utilisé un mélange 90:10 d'acétate d'éthyle:méthanol comme éluant.

### 5.8.1. Sel trifluoroacétique de 7-N-(β-alanyl)amino-1,2-diméthylphénoxazin-3-one

On a procédé comme indiqué dans le point 5.8. ci-dessus à partir de 0,047 g (0,1138 mol) de 7-*N*-(*N*'-*t*-*butoxycarbonyl*-β-alanyl)amino-1,2-diméthylphénoxazin-3-one. On a obtenu le produit du titre sous forme d'un solide rouge (0,046 g, 95%).

### 5.8.2. Sel trifluoroacétique de 7-N-(β-alanyl)amino-1,2,4-triméthylphénoxazin-3-one

On a procédé comme indiqué dans le point 5.8. ci-dessus à partir de 0,081 g (0,1897 mmol) de 7-*N*-(N'-t-butoxycarbonyl-β-alanyl)amino-1,2,4-triméthylphénoxazin-3-one. On a obtenu le produit du titre sous forme d'un solide rouge (0,080 g, 96%).

### Exemple 6 : Préparation de la 7-N-(N-'-t-butoxycarbonyl-β-alanyl)amino-1-méthyl-2-chlorophénoxazin-3-one-4-carboxylate de méthyle

On a dissous 1,76 g (10 mmol) de 1,4-dichlorobenzoquinoneimine dans 50 ml d'éthanol absolu en agitant doucement A cette solution agitée, on a ajouté 1,82 g (10 mmol) de 4-méthyl-2,6-dihydroxybenzoate de méthyle. On a chauffé doucement au reflux la solution agitée jusqu'au point où un exotherme important est apparu, nécessitant le retrait du flacon de la source de chaleur. Après diminution de l'exothermie, on a porté au reflux le mélange réactionnel pour 30 minutes supplémentaires et on a laissé refroidir à la température ambiante. Après avoir laissé 3 heures de plus à la température ambiante, on a recueilli le produit solide par filtration par aspiration, on l'a lavé avec un peu d'eau chaude et on a aspiré pour l'obtenir aussi sec que possible avant séchage dans un dessiccateur sous vide. On a soumis le résidu à une chromatographie sur couche mince sur gel de silice avec de l'acétate d'éthyle comme phase mobile. On a observé une quantité considérable de produit de base à couleur foncée, ainsi que le composant rose fluorescent. On a dissous le produit solide dans de l'acétate d'éthyle, on l'a filtré et on l'a fait passer au travers d'un cône de gel de silice. Le filtrat est essentiellement dénué de produit de base. On a retiré le solvant sous pression réduite et on a isolé le produit solide.

On a soumis le produit solide à une aminoacylation en utilisant de la *t*-*BOC*-β-alanine, comme décrit précédemment, pour obtenir le composé du titre.

### Exemple 7 : Synthèse de la 7-N-(N'-t-butoxycarbonyl-β-alanyl)amino-6-méthyl-phénoxazin-3-one

### 7.1. Synthèse de la N-acétyl-2-méthyl-3-fluoroaniline

A une solution de 3,161 g (25,26 mmol de 2-méthyl-3-fluoroaniline et de 3,87 ml (27,78 mmol) de triéthylamine dans 50 ml de DCM à 0°C, on a ajouté 1,98 ml (27,78 mmol) de chlorure d'acétyle tout en agitant. On a laissé la solution se réchauffer à la température ambiante et on l'a agitée pendant 1 heure. On a lavé la solution avec de l'eau (3 fois 50 ml), on l'a séché avec du MgSO₄ et on a retiré le solvant sous pression réduite. Après recristallisation dans un mélange d'huile minérale/acétate d'éthyle (EtOac), on a obtenu 3,844 g (91 %) du composé du titre sous forme de cristaux blancs.

### 7.2. Synthèse de la N-acétyl-2-méthyl-3-fluoro-4-nitroaniline

On a mis à réagir le composé obtenu dans le point 7.1. ci-dessus avec un mélange de 5 ml d'acide sulfurique concentré et de 5 ml d'acide nitrique dans 10 ml d'acide acétique à 18°C pendant 1 heure. On a ensuite dilué la solution réactionnelle dans 100 ml d'eau et on l'a extrait dans de l'acétate d'éthyle (EtOAc) (3 fois 50 ml), on l'a séchée sur du MgSO₄ et on a retiré le solvant sous vide. On a isolé le composé du titre sous forme de solide blanc (1,96 g, 71%).

### 7.3. Synthèse de la 2-méthyl-3-fluoro-4-nitroaniline

On a porté au reflux 1,311 g (6,18 mmol) du composé obtenu dans le point 7.2. ci-dessus dans de l'acide chlorhydrique 5M pendant 2 heures. On a neutralisé la solution avec du carbonate de sodium, puis on l'a extraite avec du diéthyléther (3 fois 50 ml), on l'a séchée sur du MgSO₄ et on a retiré le solvant sous vide. On a purifié le résidu par chromatographie sur colonne en utilisant un mélange 70:30 d'essence minérale:EtOAc comme éluant. On a isolé le produit du titre sous forme d'un solide jaune (0,589 g, 94 %).

### 7.4. Synthèse du 2-fluoro-3,6-dinitrotoluène

On a ajouté goutte à goutte une solution de 0,357 g (2,10 mmol) du composé obtenu dans le point 7.3. ci-dessus dans 4 ml d'acide acétique, à une solution de 1,61 g (10,46 mmol) de perborate sodique tétrahydraté dans 1 ml de EtOAc, à 65°C, et on a agité le mélange pendant 6 heures. On a dilué la solution réactionnelle dans 50 ml d'eau, on l'a extraite dans du diéthyléther (3 fois 20 ml), on l'a séchée sur du MgSO₄ et on a retiré le solvant sous vide. On a purifié le résidu par chromatographie sur colonne en utilisant un mélange 70:30 d'essence minérale:trichlorométhane comme éluant pour obtenir le produit du titre sous forme de liquide jaune (0,274 g, 65%).

### 7.5 Synthèse du 2,5-diméthoxyphénol

On a procédé comme indiqué dans le point 5.3. ci-dessus à partir de 5,53 g (0,0333 mol) de 2,5-diméthoxybenzaldéhyde. On a isolé le produit du titre sous forme d'huile jaune en utilisant un mélange 80:20 d'essence minérale légère (60-80°C):diéthyléther comme éluant (4,27 g, 83%).

### 7.6 Synthèse de 1-(3',6'-dinitro-2'-méthylphénoxy)-2,5-diméthoxybenzène

On a procédé comme indiqué dans le point 5.4. ci-dessus à partir de 0,366 g (1,83 mmol) du 2-fluoro-3,6-dinitrotoluène préparé dans le point 7.4. ci-dessus et 0,282 g (1,83 mmol) de 2,5-diméthoxyphénol préparé dans le point 7.5. ci-dessus. On a isolé le produit du titre sous forme de solide jaune en utilisant un mélange 70:30 d'essence minérale légère (60-80°C):diéthyléther comme éluant (0,400 g, 65,5%).

### 7.7. Synthèse du composé du titre

On peut obtenir le composé du titre comme indiqué dans les points 5.5 à 5.7. ci-dessus.

### Exemple 8 : Détection de l'activité L-alanine peptidase des bactéries à Gram négatif

On a utilisé les composés, chloré ou non, préparés dans l'exemple 1, à savoir la 7-N-(*N'-t-butoxycarbonyl*-L-alanyl)amino-2-chloro-1-pentylphénoxazin-3-one (Composé L-alanyl chloré) et la 7-N-(*N*'-*t*-*butoxycarbonyl*-L-alanyl)amino-1-pentylphénoxazin-3-one (Composé L-alanyl non chloré), lesquels ont été déprotégés selon le mode opératoire décrit dans l'exemple 3.

On a dissous 10 mg de chacun des composés L-alanyle dans 1 ml de diméthylsulfoxyde, et on a ajouté le mélange à 200 ml d'agar Columbia fondu à 50°C. Le milieu ainsi constitué a été réparti dans des boîtes de Petri (concentration finale de chaque substrat : 50 mg/l).

On a ensuite ensemencé, à l'oese calibrée 10 µl, sur chaque milieu, des souches de collections internationales ou issues de la collection de la Demanderesse, à partir de suspensions calibrées de 0,5 McFarland. Toutes les souches ont également été inoculées sur des milieux d'agar Columbia sans substrat chromogénique comme témoin de croissance. Toutes les cultures ont été incubées de 24 à 48 h à 37°C.

Les résultats de croissance et de coloration obtenus entre 24 et 48 h d'incubation sont donnés dans le tableau 1, dans lequel C signifie croissance, Co signifie couleur, I signifie incolore, le sigle ++ signifie très bonne croissance, le sigle + signifie bonne croissance de la souche, le sigle +/- signifie croissance moyenne de la souche et le sigle - signifie absence de croissance de la souche.

**Tableau 1**

| | | | **Composé L-alanyl non chloré** | | **Composé L-alanyl chloré** | | **Témoin** |
|---|---|---|---|---|---|---|---|
| **N°** | **Nom de la souche** | **N° d'accès international** | **Co** | **C** | **Co** | **C** | **C** |
| 1 | ***Escherichla coli Klebsiella*** | NCTC 10418 | pourpre | + | Rose/Poupre | + | + |
| 2 | ***pneumoniae Salmonella*** | NCTC 10896 | pourpre | + | Rose/Poupre | + | + |
| 3 | ***hadar*** | | pourpre | + | Rose/Poupre | + | + |
| 4 | ***Proteus mirabilis Staphylococcus*** | NCTC 10975 | pourpre | + | Rose/Poupre | + | + |
| 5 | ***aureus*** | NCTC 6571 | I | - | I | + | + |
| 6 | ***Providencia rettgeri*** | NCTC 7475 | pourpre | + | Rose/Poupre | + | + |
| 7 | ***Pseudomonas aeruginosa*** | NCTC 10662 | pourpre | + | Rose/Poupre | + | ++ |
| 8 | ***Listeria monocytogenes*** | NCTC 11994 | I | - | I | + | + |
| 9 | ***Streptococcus pyogenes*** | NCTC 8306 | I | - | I | +/- | +/- |

Les résultats dans le tableau 1 ci-dessus montrent que le substrat enzymatique de l'invention permet la détection de toutes les souches de bactéries car elles croissent toutes, mais qu'on observe une modification de coloration uniquement pour les souches à Gram - (souches 1 à 4 et 6 à 7), ce qui permet également de faire une discrimination entre les souches à Gram - et les souches à Gram +.

### Exemple 9 : Détection de l'activité β-alanine peptidase des bactéries du genre Pseudomonas aeruginosa

Pour ce faire, on a utilisé les composés de β-alanine, chloré ou non, préparés dans l'exemple 2, à savoir la 7-N-(*N*'-*t-butoxycarbonyl*-β-alanyl)amino-2-chloro-1-pentylphénoxazin-3-one (composé béta-alanyl chloré) et la 7-N-(*N'-t-butoxycarbonyl-*β-alanyl)amino-1-pentylphénoxazin-3-one (composé béta-alanyl non chloré), lesquels ont été déprotégés selon le mode opératoire décrit dans l'exemple 5.

On a également préparé les boîtes de Petri et on a inoculé les souches selon le protocole décrit dans l'exemple 5.

Les résultats de coloration entre 24 et 48 h d'incubation sont indiqués dans le tableau 2 ci-après.

**Tableau 2**

| **Nom de la souche** | **N° d'accès international** | **Composé béta-alanyl non chloré** | **Composé béta-alanyl chloré** |
|---|---|---|---|
| ***Pseudomonas aeruginosa*** | | pourpre | pourpre |
| ***Pseudomonas aeruginosa*** | | pourpre | pourpre pale |
| ***Pseudomonas aeruginosa*** | | pourpre | pourpre pale |
| ***Pseudomonas aeruginosa*** | | pourpre | pourpre |
| ***Burkholderia cepacia GI*** | LMG1222 | jaune | jaune |
| ***Burkholderia cepacia GIII*** | LMG 18832 | jaune | jaune |
| ***Acinetobacter baumannii*** | ATCC 19606 | incolore | incolore |
| ***Acinetobacter calcoaceticus*** | | incolore | incolore |
| ***Pseudomonas fragilis*** | NCIMB 8987 | incolore | incolore |
| ***Pseudomonas maltophilla*** | | incolore | incolore |
| ***Ralstonia basilensis*** | | incolore | incolore |
| ***Ralstonia taiwanensis*** | | incolore | incolore |

Les résultats dans le tableau 2 ci-dessus mettent en évidence que les composés de l'invention permettent de détecter préférentiellement les bactéries *Pseudomonas aeruginosa* (développement d'une coloration pourpre pale à pourpre).

### Exemple 10 : Détection de l'activité pyroglutamyl-peptidase

Pour ce faire, on a utilisé les composés de pyroglutamyl, chloré ou non, préparés dans l'exemple 4, à savoir la 7-N-(L-pyroglutamyl)amino-2-chloro-1-pentylphénoxazin-3-one (composé L-pyroglutamyl chloré) et la 7-N-(L-pyroglutamyl)amino-1-pentylphénoxazin-3-one (composé L-pyroglutamyl non chloré), lesquels ont été déprotégés selon le mode opératoire décrit dans l'exemple 5.

On a également préparé les boîtes de Petri et on a inoculé les souches selon le protocole décrit dans l'exemple 5.

Les résultats de coloration entre 24 et 48 h d'incubation sont indiqués dans le tableau 3 ci-après.

**Tableau 3**

| **Nom de la souche** | **N° d'accès international** | **Composé pyroglutamyl non chloré** | **Composé pyroglutamyl chloré** |
|---|---|---|---|
| ***Pseudomonas aeruginosa*** | | pourpre | pourpre |
| ***Pseudomonas aeruginosa*** | | pourpre | pourpre |
| ***Pseudomonas aeruginosa*** | | pourpre | pourpre |
| ***Burkholderia cepacia GI*** | LMG1222 | jaune | jaune |
| ***Burkholderia cepacia GIII*** | LMG 18832 | jaune | jaune |
| ***Acinetobacter baumannii*** | ATCC 19606 | incolore | incolore |
| ***Acinetobacter calcoaceticus*** | | incolore | incolore |
| ***Pseudomonas fluorescens*** | | incolore | incolore |
| ***Pseudomonas fragilis*** | NCIMB 8987 | incolore | incolore |
| ***Pseudomonas maltophilia*** | | incolore | incolore |
| ***Ralstonia basilensis*** | | incolore | incolore |
| ***Ralstonia taiwanensis*** | | incolore | incolore |

Les résultats dans le tableau 3 ci-dessus mettent en évidence que les composés de l'invention permettent de détecter préférentiellement les bactéries *Pseudomonas aeruginosa.*

### Exemple 11 : Détection de l'activité β-alanine-peptidase des bactéries du genre Pseudomonas aeruginosa

Pour ce faire, on a utilisé les composés de β-alanine préparés dans les exemples 5 et 6, à savoir la 7-*N*-(N'-t-butoxycarbonyl-β-alanyl)amino-1,2-diméthylphénoxazin-3-one (b-ala-DMP), la 7-*N*-(*N'-t-butoxycarbonyl*-β-alanyl)amino-1,2,4-triméthylphénoxazin-3-one (b-ala-TMP) et 7-N-(*N'-t-butoxycarbonyl*-β-alanyl)amino-1-méthyl-2-chloro-4-(oxo-1-méthyl)phénoxazin-3-one (b-ala-MCMP), lesquels ont été déprotégés comme décrit dans les exemples précédents.

On a également préparé les boîtes de Petri et on a inoculé les souches selon le protocole décrit dans l'exemple 7. Les résultats de coloration entre 24 et 48 h d'incubation sont indiqués dans le tableau 4 ci-après.

**Tableau 4**

| **Nom de la souche** | **N° d'accès international** | **b-ala-DMP** | **b-ala-TMP** | **b-ala-MCMP** |
|---|---|---|---|---|
| ***Pseudomonas aeruginosa*** | ATCC 10145 | Rose intense | Rose orangé | Violet |
| ***Pseudomonas aeruginosa*** | ATCC 27853 | Rose | Rose | Violet |
| ***Pseudomonas aeruginosa*** | * | Rose intense | Rose orangé | Violet |
| ***Pseudomonas aeruginosa*** | ATCC 9027 | Rose intense | Rose | Rose-violet |
| ***Pseudomonas aeruginosa*** | * | Incolore | Incolore | Incolore |
| ***Pseudomonas fluorescents*** | * | Incolore | Incolore | Incolore |
| ***Pseudomonas putida*** | * | Incolore | Incolore | Incolore |
| ***Burkholderia cepacia*** | LMG 18941 | Rose pale | Rose pale | Incolore |
| ***Burkholderia Gladioli*** | LGM 6880 | Incolore | Incolore | Incolore |
| ***Escherichia coli*** | * | Orange | Orange | Incolore |

### * Collection de la Demanderesse

Les résultats dans le tableau 4 ci-dessus mettent en évidence que les composés de l'invention permettent de détecter préférentiellement les bactéries *Pseudomonas aeruginosa* (développement d'une coloration rose à violette). On peut également noter que les résultats peuvent varier en termes de couleur, intensité de couleur, de même qu'en termes de sensibilité et spécificité vis-à-vis de l'espèce *Pseudomonas aeruginosa,* selon les substituants utilisés et leur position sur le noyau principal, de sorte qu'on peut envisager d'utiliser ces différents substrats dans des applications particulières différentes, selon l'objectif visé.

## Revendications

1. Substrat enzymatique chromogénique, **caractérisé en ce qu'**il répond à la formule (I) suivante : 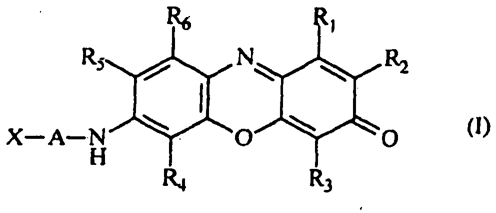 dans laquelle
- R₁ représente un atome d'hydrogène, un groupement alkyle en C₁-C₁₂, un groupement aralkyle en C₆-C₁₄, un groupement aryle, -COOH, -COOR' ou -NR"R"',
- R₂ représente un atome d'hydrogène, un atome d'halogène, un groupement alkyle en C₁-C₁₂, -COOH ou -COOR', étant entendu qu'au moins un parmi R₁ et R₂ est un atome d'hydrogène ou un atome d'halogène,
- R₃ représente un atome d'hydrogène, un atome d'halogène, -CN, -CONH₂, -COOR' ou -COR',
- R₄, R₅ et R₆, chacun indépendamment, représentent un atome d'hydrogène ou un groupement alkyle en C₁-C₃, étant entendu qu'au moins un parmi R₄, R₅ et R₆ est un atome d'hydrogène,
- R' représente un atome d'hydrogène ou un groupement alkyle en C₁-C₆,
- R" et R"' représentent chacun indépendamment un groupement alkyle en C₁-C₆, ou bien R" et R"', ensemble avec l'atome d'azote auquel ils sont liés, forment un noyau hétérocyclique contenant un ou plusieurs hétéroatomes,
- A représente au moins un acide aminé et
- X représente un agent de blocage ou rien.

2. Substrat enzymatique chromogénique selon la revendication 1, **caractérisé en ce que** R₁ représente un groupement alkyle, de préférence en C₃-C₆, et R₂ représente un atome d'hydrogène.

3. Substrat enzymatique chromogénique selon la revendication 1, **caractérisé en ce que** R₁ représente un atome d'hydrogène et R₂ représente un groupement alkyle, de préférence un groupement éthyle ou hexyle.

4. Substrat enzymatique chromogénique selon l'une quelconque des revendications 1 à 3, **caractérisé en ce que** R₃, R₄, R₅ et R₆ représentent un atome d'hydrogène.

5. Milieu réactionnel comprenant au moins un substrat chromogénique enzymatique, tel que défini dans l'une quelconque des revendications 1 à 4, seul ou en combinaison avec au moins un autre substrat enzymatique spécifique d'une activité enzymatique différente de l'activité peptidase détectée par ledit substrat tel que défini dans l'une quelconque des revendications 1 à 4.

6. Milieu selon la revendication 5, **caractérisé en ce qu'**il est un milieu de culture.

7. Milieu selon l'une des revendications 5 ou 6, **caractérisé en ce qu'**il est sous forme gélifiée.

8. Utilisation d'un substrat enzymatique chromogénique tel que défini dans l'une quelconque des revendications 1 à 4, ou d'un milieu réactionnel tel que défini dans l'une quelconque des revendications 5 à 7, pour la détection et/ou l'identification et/ou la quantification *in vitro* des micro-organismes exprimant au moins une activité peptidase.

9. Procédé pour la détection et/ou l'identification et/ou la quantification des micro-organismes exprimant au moins une activité peptidase, **caractérisé en ce qu'**il consiste à :
• disposer d'un milieu réactionnel, tel que défini dans l'une quelconque des revendications 5 à 7,
• ensemencer le milieu avec un échantillon biologique à tester,
• laisser incuber, et
• révéler la présence d'au moins une activité peptidase seule ou en combinaison avec au moins une autre activité enzymatique différente de cette même activité peptidase.

10. Procédé pour la différentiation chez des bactéries de leur appartenance aux germes à Gram positif ou aux germes à Gram négatif, **caractérisé en ce qu'**il consiste à :
• disposer d'un milieu réactionnel, tel que défini dans l'une quelconque des revendications 5 à 7 et dans lequel le substituant A du substrat chromogénique est la L-alanine,
• ensemencer le milieu avec un échantillon biologique à tester,
• laisser incuber, et
• révéler la présence d'au moins une variation de coloration synonyme de la présence de germe(s) à Gram négatif.

11. Procédé pour la détection de *Pseudomonas aeruginosa,* **caractérisé en ce qu'**il consiste à :
• disposer d'un milieu réactionnel, tel que défini dans l'une quelconque des revendications 5 à 7 et dans lequel le substituant A du substrat chromogénique est la β-alanine ou la pyroglutamine,
• ensemencer le milieu avec un échantillon biologique à tester,
• laisser incuber, et
• révéler la présence d'au moins une variation de coloration synonyme de la présence de germe(s) de *Pseudomonas aeruginosa.*

## Claims

1. A chromogenic enzymatic substrate, **characterized in that** it corresponds to formula (I) below: 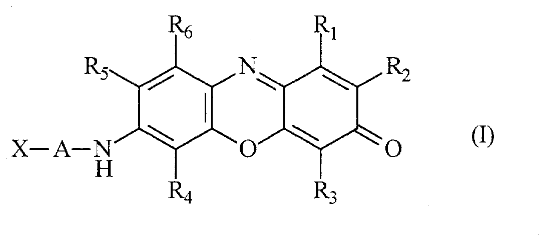 in which
- R₁ represents a hydrogen atom, a C₁-C₁₂ alkyl group, a C₆-C₁₄ aralkyl group, an aryl group, -COOH, -COOR' or -NR"R"',
- R₂ represents a hydrogen atom, a halogen atom, a C₁-C₁₂ alkyl group, -COOH or
- COOR', it being understood that at least one of R₁ and R₂ is a hydrogen atom or a halogen atom,
- R₃ represents a hydrogen atom, a halogen atom, -CN, -CONH₂, -COOR' or -COR',
- R₄, R₅ and R₆ each independently represent a hydrogen atom or a C₁-C₃ alkyl group, it being understood that at least one of R₄, R₅ and R₆ is a hydrogen atom,
- R' represents a hydrogen atom or a C₁-C₆ alkyl group,
- R" and R"' each independently represent a C₁-C₆ alkyl group, or else R" and R"', together with the nitrogen atom to which they are attached, form a heterocyclic ring containing one or more heteroatoms,
- A represents at least one amino acid, and
- X represents a blocking agent or nothing.

2. The chromogenic enzymatic substrate as claimed in claim 1, **characterized in that** R₁ represents an alkyl, preferably C₃-C₆, group, and R₂ represents a hydrogen atom.

3. The chromogenic enzymatic substrate as claimed in claim 1, **characterized in that** R₁ represents a hydrogen atom and R₂ represents an alkyl group, preferably an ethyl or hexyl group.

4. The chromogenic enzymatic substrate as claimed in any one of claims 1 to 3, **characterized in that** R₃, R₄, R₅ and R₆ represent a hydrogen atom.

5. A reaction medium comprising at least one chromogenic enzymatic substrate as defined in any one of claims 1 to 4, alone or in combination with at least one other enzymatic substrate specific for an enzymatic activity other than peptidase activity detected by said substrate as defined in any one of claims 1 to 4.

6. The medium as claimed in claim 5, **characterized in that** it is a culture medium.

7. The medium as claimed in either of claims 5 or 6, **characterized in that** it is in gelled form.

8. The use of a chromogenic enzymatic substrate as defined in any one of claims 1 to 4, or of a reaction medium as defined in any one of claims 5 to 7, for detecting and/or identifying and/or quantifying, *in vitro,* microorganisms expressing at least one peptidase activity.

9. A method for detecting and/or identifying and/or quantifying microorganisms expressing at least one peptidase activity, **characterized in that** it consists in :
• providing a reaction medium, as defined in any one of claims 5 to 7,
• inoculating the medium with a biological sample to be tested,
• leaving this to incubate, and
• revealing the presence of at least one peptidase activity alone or in combination with at least one other enzymatic activity other than this same peptidase activity.

10. A method for differentiating bacteria in terms of whether they belong to Gram-positive microorganisms or to Gram-negative microorganisms, **characterized in that** it consists in :
• providing a reaction medium, as defined in any one of claims 5 to 7 and in which the substituent A of the chromogenic substrate is L-alanine,
• inoculating the medium with a biological sample to be tested,
• leaving this to incubate, and
• revealing the presence of at least one color variation synonymous with the presence of Gram-negative microorganism(s).

11. A method for detecting *Pseudomonas aeruginosa,* **characterized in that** it consists in :
• providing a reaction medium, as defined in any one of claims 5 to 7 and in which the substituent A of the chromogenic substrate is β-alanine or pyroglutamine,
• inoculating the medium with a biological sample to be tested,
• leaving this to incubate, and
• revealing the presence of at least one color variation synonymous with the presence of *Pseudomonas aeruginosa* microorganism(s).

## Patentansprüche

1. Chromogenes Enzymsubstrat, **dadurch gekennzeichnet, dass** es der folgenden Formel (I) 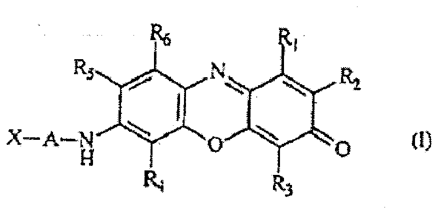 mit den folgenden Bedeutungen entspricht:
- R₁ bedeutet ein Wasserstoffatom, eine C₁-C₁₂-Alkylgruppe, eine C₆-C₁₄-Aralkylgruppe, eine Arylgruppe, -COOH, -COOR' oder -NR"R"',
- R₂ bedeutet ein Wasserstoffatom, ein Halogenatom, eine C₁-C₁₂-Alkylgruppe, -COOH oder -COOR', mit der Maßgabe, dass mindestens eines von R₁ und R₂ ein Wasserstoffatom oder ein Halogenatom bedeutet,
- R₃ bedeutet ein Wasserstoffatom, ein Halogenatom, -CN, -CONH₂, -COOR' oder -COR',
- R₄, R₅ und R₆ bedeuten jeweils unabhängig voneinander ein Wasserstoffatom oder eine C₁-C₃-Alkylgruppe, mit der Maßgabe, dass mindestens eines von R₄, R₅ und R₆ ein Wasserstoffatom bedeutet,
- R' bedeutet ein Wasserstoffatom oder eine C₁-C₆-Alkylgruppe,
- R" und R"' bedeuten jeweils unabhängig voneinander eine C₁-C₆-Alkylgruppe, oder R" und R"' gemeinsam mit dem Stickstoffatom, an das sie gebunden sind, bilden einen heterocyclischen Kern, der ein oder mehrere Heteroatome enthält,
- A bedeutet mindestens eine Aminosäure und
- X bedeutet ein Blockierungsmittel oder gar nichts.

2. Chromogenes Enzymsubstrat nach Anspruch 1, **dadurch gekennzeichnet, dass** R₁ eine Alkylgruppe, vorzugsweise eine C₃-C₆-Alkylgruppe, bedeutet und R₂ ein Wasserstoffatom bedeutet.

3. Chromogenes Enzymsubstrat nach Anspruch 1, **dadurch gekennzeichnet, dass** R₁ ein Wasserstoffatom bedeutet und R₂ eine Alkylgruppe, vorzugsweise eine Ethyl- oder Hexylgruppe, bedeutet.

4. Chromogenes Enzymsubstrat nach einem der Ansprüche 1 bis 3, **dadurch gekennzeichnet, dass** R₃, R₄, R₅ und R₆ ein Wasserstoffatom bedeuten.

5. Reaktionsmedium, umfassend mindestens ein chromogenes Enzymsubstrat wie in einem der Ansprüche 1 bis 4 definiert, und zwar allein oder in Kombination mit mindestens einem anderen Enzymsubstrat, das für eine Enzymaktivität spezifisch ist, die sich von der Peptidaseaktivität unterscheidet, und von dem Substrat, die wie in einem der Ansprüche 1 bis 4 definiert ist, nachgewiesen.

6. Medium nach Anspruch 5, **dadurch gekennzeichnet, dass** es sich um ein Kulturmedium handelt.

7. Medium nach einem der Ansprüche 5 oder 6, **dadurch gekennzeichnet, dass** es in gelierter Form vorliegt.

8. Verwendung eines chromogenen Enzymsubstrats wie in einem der Ansprüche 1 bis 4 definiert oder eines Reaktionsmediums wie in einem der Ansprüche 5 bis 7 definiert zum Nachweisen und/oder Identifizieren und/oder quantitativem Bestimmen *in vitro* von Mikroorganismen, die mindestens eine Peptidaseaktivität exprimieren.

9. Verfahren zum Nachweisen und/oder Identifizieren und/oder quantitativem Bestimmen von Mikroorganismen, die mindestens eine Peptidaseaktivität exprimieren, **dadurch gekennzeichnet, dass** es aus Folgendem besteht:
• Verfügen über ein Reaktionsmedium wie in einem der Ansprüche 5 bis 7 definiert,
• Inokulieren des Mediums mit einer zu prüfenden biologischen Probe,
• Inkubieren lassen und
• Aufzeigen des Vorliegens von mindestens einer Peptidaseaktivität, allein oder in Kombination mit mindestens einer weiteren Enzymaktivität, die sich von eben dieser Peptidaseaktivität unterscheidet.

10. Verfahren zum Differenzieren bei Bakterien, ob es sich um grampositive Mikroorganismen oder um gramnegative Mikroorganismen handelt, **dadurch gekennzeichnet, dass** es aus Folgendem besteht:
• Verfügen über ein Reaktionsmedium wie in einem der Ansprüche 5 bis 7 definiert, und in dem es sich bei dem Substituenten A des chromogenen Substrats um L-Alanin handelt,
• Inokulieren des Mediums mit einer zu prüfenden biologischen Probe,
• Inkubieren lassen und
• Aufzeigen des Vorliegens von mindestens einer Farbvariation, die dem Vorliegen von (einem) gramnegativen Mikroorganismus/Mikroorganismen entspricht.

11. Verfahren zum Nachweisen von *Pseudomonas aeruginosa,* **dadurch gekennzeichnet, dass** es aus Folgendem besteht:
• Verfügen über ein Reaktionsmedium wie in einem der Ansprüche 5 bis 7 definiert, und in dem es sich bei dem Substituenten A des chromogenen Substrats um β-Alanin oder Pyroglutamin handelt,
• Inokulieren des Mediums mit einer zu prüfenden biologischen Probe,
• Inkubieren lassen und
• Aufzeigen des Vorliegens von mindestens einer Farbvariation, die dem Vorliegen eines Pseudomonas-aeruginosa-Mikroorganismus bzw. von Pseudomonas-aeruginosa-Mikroorganismen entspricht.
